**Europäisches Patentamt**

**European Patent Office**

**Office européen des brevets**

(11) Numéro de publication : **0 506 519 A1**

(12) **DEMANDE DE BREVET EUROPEEN**

(21) Numéro de dépôt : **92400725.5**

(22) Date de dépôt : **18.03.92**

(51) Int. Cl.$^5$ : **G01N 33/00**

(30) Priorité : **19.03.91 FR 9103340**

(43) Date de publication de la demande :
**30.09.92 Bulletin 92/40**

(84) Etats contractants désignés :
**AT BE CH DE DK ES FR GB GR IT LI LU NL PT SE**

(71) Demandeur : **SOLLAC**
**Immeuble Elysées La Défense, 29 Le Parvis**
**F-92800 PUTEAUX (FR)**

(72) Inventeur : **Tosin, René**
**2, rue Adjudant Fonvieille, St. Pierre 2**
**F-13700 Marignane (FR)**

(74) Mandataire : **Lanceplaine, Jean-Claude et al**
**CABINET LAVOIX 2, Place d'Estienne d'Orves**
**F-75441 Paris Cédex 09 (FR)**

(54) **Dispositif de conditionnement de gaz industriels pour analyse en ligne.**

(57)    La présente invention a pour objet un dispositif (1) de conditionnement de gaz industriels chargés en impuretés, notamment de gaz de cokerie et de hauts fourneaux en vue de leur analyse.

Le dispositif comporte une arrivée (2) d'air comprimé, des moyens (3) de régulation et d'alimentation en air comprimé, des moyens (4) de diffusion de l'air comprimé, une arrivée (5a) de gaz industriels, au moins un échangeur de chaleur air-gaz (6) pour le refroidissement et la purification des gaz industriels et une sortie (5b) des gaz industriels conditionnés.

FIG.1

EP 0 506 519 A1

La présente invention concerne un dispositif de conditionnement de gaz industriels chargés en impuretés, notamment de gaz de cokerie et de hauts fourneaux, en vue de leur analyse en ligne.

On sait que dans les aciéries, l'élaboration des produits sidérurgiques s'effectue selon des processus de fabrication dont la conduite est assurée par un calculateur.

En particulier, dans la filière de production de la fonte, il est indispensable de connaître en permanence la composition exacte des gaz pour la conduite automatisée du processus.

Dans des batteries de four à coke, la composition des gaz mélangés de coke et de hauts fourneaux doit être déterminée en continu et de la manière la plus précise possible pour en déduire le pouvoir calorifique inférieur du gaz, cette valeur permettant de réguler la température de chauffage des batteries.

Or, les batteries de four émettent des fumées sur lesquelles on procède à une analyse en ligne dans le but de déterminer le degré d'avancement de la combustion.

L'analyse des gaz intervient également dans des dégoudronneurs en tant que système de sécurité.

Dans ces différentes installations, on effectue un dépoussiérage électrostatique des gaz de cokerie fortement chargés en impuretés. A cet effet, les gaz traversent une chambre équipée d'électrodes entre lesquelles règne une tension de l'ordre de 55kV, aussi, dès que la teneur en oxygène dépasse 0,5%, le risque d'apparition d'étincelles est élevé et l'on doit couper le champ électrostatique.

L'analyse préalable des gaz permet donc de s'assurer qu'ils ne contiennent pas une teneur en oxygène trop proche de cette valeur limite.

Avant l'analyse, il est également nécessaire de procéder au conditionnement préalable des gaz industriels prélevés en ligne qui sont chargés en impuretés et, notamment, des gaz de cokerie contenant des constituants divers : eau, sulfure d'hydrogène, naphtalène, acide cyanhydrique, goudron, poussières et ammoniac.

Généralement, les analyses de gaz connues reposent sur le principe de l'absorption du gaz dans le rayonnement infrarouge. Or, cette propriété est affectée d'une double sensibilité liée à la présence de vapeur d'eau dans les échantillons d'analyse.

En effet, certaines bandes d'absorption de la vapeur interfèrent avec celles du gaz à analyser si bien qu'il en résulte une erreur de sélectivité des mesures.

De plus, les appareils utilisant ce principe se comportent comme des analyseurs volumétriques, c'est à dire que la présence d'un composant supplémentaire dans le gaz fausse les mesures.

Par conséquent, on comprend aisément qu'il faut piéger l'eau présente dans l'échantillon de gaz sous forme de vapeur et de liquide, mais également éliminer les matières en suspension et toutes les impuretés pouvant occasionner un disfonctionnement, voire une destruction des analyseurs.

Outre le fait que le gaz doit être épuré, il est nécessaire de le refroidir pour l'amener à une température voisine de 20° à 25°C en entrée d'analyseur.

Dans le domaine de la conduite de processus industriels, notamment de l'élaboration des aciers, l'analyse en ligne des gaz industriels chargés en impuretés doit fournir des résultats d'une grande fiabilité et avec un temps de réponse aussi court que possible afin que les mesures effectuées soient transmises rapidement au calculateur qui agira en conséquence sur le processus.

Or, les conditionneurs de gaz existant sur le marché, en particulier les groupes frigorifiques hermétiques et les assécheurs à effet Peltier présentent des inconvénients majeurs.

Les groupes frigorifiques ou réfrigérants électriques ne permettent pas une bonne analyse car l'asséchage est insuffisant et, de ce fait, l'excès d'eau entraîne des particules qui perturbent les cellules des analyseurs.

Par ailleurs, ils s'encrassent très rapidement compte tenu de la forte teneur en impuretés des gaz, ce qui conduit inévitablement à un arrêt du système de prélèvement en ligne des échantillons de gaz et à des travaux de maintenance.

Dans le cas des assécheurs à effet Peltier, l'échantillon de gaz prélevé parcourt une chambre équipée de chicanes qui sont réalisées dans un matériau à haute conductibilité thermique, ladite chambre étant calorifugée par un enrobage de mousse synthétique.

Au contact de cette chambre est disposée une face froide de deux thermo-éléments, permettant ainsi au gaz de se refroidir par échanges thermiques.

Etant donné que cet appareil retient l'eau sous forme de givre, il s'obstrue régulièrement. Par conséquent deux chambres identiques sont prévues afin que, lorsque l'une d'elles est entièrement givrée, un combinateur cyclique agissant sur des électrovannes commande un basculement sur l'autre chambre.

Un tel assécheur comporte les inconvénients suivants.

Un piège à eau est nécessaire au fonctionnement, ce qui accroit considérablement le volume du dispositif et donc augmente le temps de réponse pour les mesures effectuées dans un analyseur situé en aval.

D'autre part, lors de la phase de dégivrage d'une des chambres, les gaz, chargés en impuretés sont dirigés au moyen d'électrovannes vers l'autre chambre et ils détériorent à cette occasion les membranes de celle-ci, ce qui provoque avec le temps des entrées d'air dans le dispositif. Cet air "neuf" perturbe les cellules des analyseurs donc fausse les mesures et endommage les analyseurs.

Durant l'arrêt de l'installation et l'intervention des équipes de maintenance sur une des chambres, on introduit une valeur fictive pour l'analyse de la composition des gaz afin que des données soient fournies en permanence au calculateur et que le processus industriel ne soit pas perturbé.

Les deux dispositifs précédemment cités ont, en outre, un inconvénient supplémentaire résidant dans le fait qu'ils utilisent un courant électrique pour leur fonctionnement, ce qui empêche leur installation dans les zones dites "sensibles" d'une aciérie où l'on doit éviter les risques d'étincelles, notamment dans les cokeries.

L'invention se propose, notamment, de remédier à ces divers inconvénients.

A cet effet, la présente invention a pour objet un dispositif de conditionnement de gaz industriels chargés en impuretés, notamment de gaz de cokerie et de hauts fourneaux, en vue de leur analyse, caractérisé en ce qu'il comporte :

– une arrivée d'air comprimé,

– des moyens de régulation et d'alimentation en air comprimé,

– des moyens de diffusion de l'air comprimé,

– une arrivée de gaz industriels chargés en impuretés,

– au moins un échangeur de chaleur air-gaz pour le refroidissement et la purification des gaz,

– et une sortie des gaz industriels conditionnés.

Selon d'autres caractéristiques de la présente invention :

– les moyens de régulation et d'alimentation en air comprimé comportent une sonde de mesure de la température des gaz et un régulateur de température relié à la sonde et à une vanne de régulation, ladite vanne étant placée en amont des moyens de diffusion de l'air,

– les moyens de régulation et d'alimentation en air comprimé comportent également au moins un filtre-détendeur, en amont de la sonde et au moins un filtre, en amont de la vanne de régulation, sur l'arrivée d'air comprimé,

– les moyens de diffusion de l'air comprimé sont formés par un vortex,

– l'échangeur de chaleur air-gaz est formé par une calandre cylindrique verticale munie à sa partie supérieure d'une bride circulaire sur laquelle est fixée une plaque de tête et par un tube en épingle muni d'ailettes, formant deux branches réunies par un coude et dans lesquelles circule l'air comprimé,

– la plaque de tête a une forme circulaire et comporte deux orifices diamétralement opposés traversant ladite plaque dans son épaisseur, chacun desdits orifices reliant deux manchettes situées de part et d'autre de la plaque, l'une desdites manchettes étant perpendiculaire à la plaque et destinée à s'engager dans une des branches du tube,

– les ailettes sont formées par une couronne sur laquelle sont également réparties sur un même diamètre des perforations, ladite couronne délimitant un évidement, chacun desdits évidements étant aligné avec celui d'une ailette immédiatement voisine, les évidement ainsi disposés définissant un espace cylindrique,

– la plaque de tête comporte un trou central aligné avec l'espace cylindrique et un manchon extérieur dans l'axe du trou, permettant ainsi l'introduction et la fixation de la sonde dans l'échangeur,

– l'échangeur comporte un tube d'injection d'eau aménagé sous la bride,

– l'échangeur comporte un récipient en partie basse, au fond duquel est pratiquée une ouverture pour la purge des condensats,

– le tube en épingle est, de préférence, en inox,

– les ailettes sont réalisées dans un matériau à forte diffusivité thermique,

– le matériau à forte diffusité thermique est, de préférence, l'aluminium.

– l'échangeur de chaleur est enveloppé d'un calorifuge,

– le calorifuge est, de préférence, en laine de verre.

La description qui va suivre permettra de mieux comprendre l'invention en se référant aux dessins annexés, donnés à titre indicatif et non limitatif, et sur lesquels :

– la Fig. 1 est une vue schématique du dispositif de conditionnement selon un mode de réalisation de l'invention,

– la Fig. 2 est une vue de face de l'échangeur du dispositif selon l'invention,

– la Fig. 3 est une vue de dessus de l'échangeur,

– la Fig. 4 est une vue en coupe longitudinale de l'ensemble tube et plaque de tête,

– la Fig. 5 est une vue de dessus de la plaque de tête,

– la Fig. 6 est une vue de dessus d'une ailette.

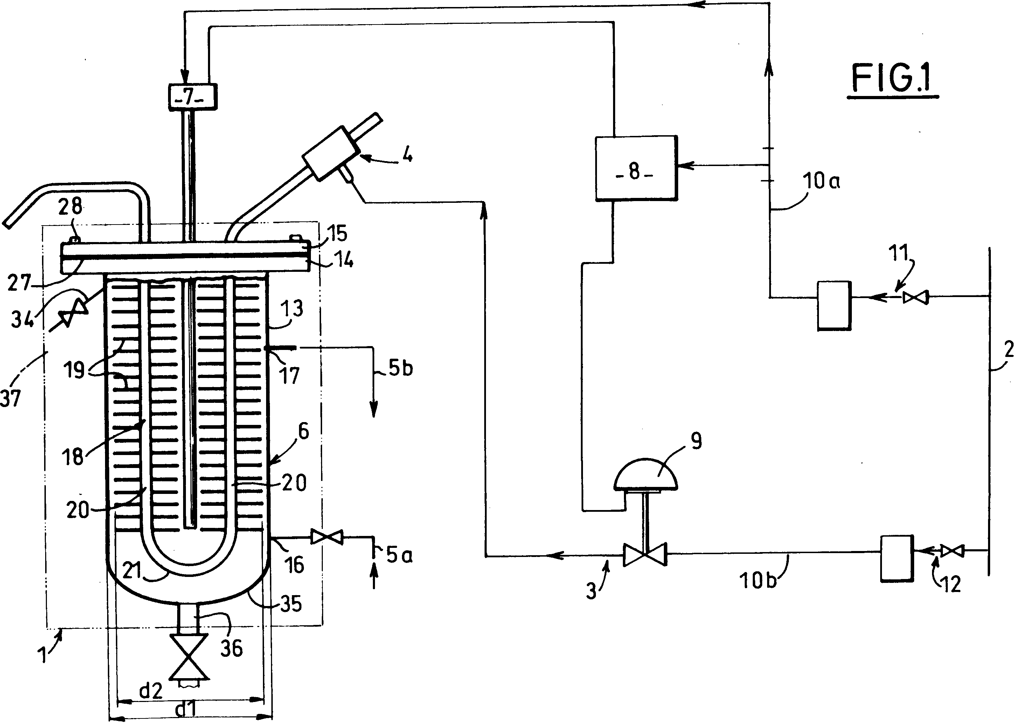

Comme représenté à la Fig. 1, le dispositif 1 de conditionnement de gaz industriels chargés en impuretés, notamment de gaz de cokerie et de hauts fourneaux, en vue de leur analyse comporte :

– une arrivée 2 d'air comprimé,

– des moyens 3 de régulation et d'alimentation en air comprimé,

– des moyens 4 de diffusion de l'air comprimé,

– une arrivée 5a de gaz industriels,

– un échangeur de chaleur air-gaz 6 pour le refroidissement et la purification des gaz industriels,

– et une sortie 5b des gaz industriels conditionnés.

Etant donné que l'on utilise de l'air comprimé dans le dispositif 1, celui-ci est rendu antidéflagrant.

Cette caractéristique essentielle de la présente invention est très avantageuse en regard des groupes frigorifiques dans lesquels le compresseur est conditionné par un moteur électrique et des assécheurs à effet Peltier utilisant également un courant électrique.

En effet, on peut envisager de placer le dispositif de conditionnement 1, en zone "explosible" et notamment à proximité des dégoudronneurs où les risques d'explosion interdisent l'emploi de systèmes dynamiques comme ceux précédemment cités dans l'état de la technique.

Par ailleurs, ces systèmes connus sont constitués de telle manière que les risques d'entrée d'air dans le circuit de gaz sont élevés du fait de la présence de raccords entre les différents éléments, de la complexité des dispositifs et de leur mise en oeuvre, et des opérations fréquentes de maintenance ce qui rend impossible leur installation en atmosphère polluée.

Le dispositif de conditionnement, appelé aussi conditionneur, selon la présente invention, a l'avantage de se présenter sous une forme compacte et simple à mettre en oeuvre permettant ainsi son installation en atmosphère polluée.

Comme représenté sur la Fig. 1, les moyens 3 de régulation et d'alimentation en air comprimé comportent une sonde 7 de mesure de la température des gaz industriels et un régulateur de température 8 relié à la sonde 7 et à une vanne de régulation 9, ladite vanne étant placée en amont des moyens 4 de diffusion de l'air comprimé.

La distribution d'air comprimé s'effectue par le biais de deux conduites, respectivement 10a et 10b, la première 10a amenant l'air à la sonde 7 et au régulateur de température 8, et la seconde 10b amenant l'air aux moyens 4 de diffusion par l'intermédiaire de la vanne de régulation 9.

Sur la première conduite 10a, en amont du régulateur de température 8 et de la sonde 7, est disposé un filtre-détendeur 11, et sur la seconde conduite 10b en amont de la vanne de régulation 9, est disposé un filtre 12.

L'utilisation d'un détendeur se justifie par le fait que les appareils de régulation fonctionnent à 1,4 bars alors que le réseau d'air comprimé fournit de l'air à une pression moyenne de 6 bars.

Les filtres 11 et 12 notamment celui placé en amont de la vanne de régulation 9, permettent d'éviter l'encrassement du circuit d'air, plus particulièrement des moyens de diffusion 4 et de l'intérieur de l'échangeur 6 par des poussières.

Ainsi, on élimine des opérations répétitives de maintenance, c'est à dire des arrêts du dispositif de conditionnement et l'on assure de meilleurs échanges thermiques au sein de cet échangeur 6.

L'encrassement est un facteur déterminant car, non seulement, il entrave l'échange des calories et donc conduit à un mauvais assèchement du gaz, une épuration insuffisante pouvant endommager les analyseurs, mais il ralentit les échanges et donc augmente le temps de réponse du dispositif, ce qui est néfaste pour les exigences d'une analyse en continu d'échantillons de gaz.

Les moyens de diffusion de l'air comprimé sont constitués par un Vortex 4 qui a pour fonction de refroidir l'air comprimé avant son entrée dans l'échangeur 6.

Cet appareil est d'emploi très simple par rapport à un groupe frigorifique électrique utilisant un fluide frigorigène, par exemple un fréon, et il n'en comporte pas les inconvénients qui sont notamment des risques de fuite, un coût élevé du fluide et une maintenance importante.

En outre, un vortex permet d'obtenir des températures en sortie de l'ordre de -20°C à -30°C, alors qu'avec un groupe frigorifique électrique, on obtient en sortie que des températures de +2°C à +12°C, ce qui limite considérablement le champ des applications.

Dans le mode de réalisation représenté à la Fig. 1, le gaz prélevé est par exemple un mélange de gaz de cokeries et de hauts fourneaux et sa température est de 40°C en entrée de l'échangeur.

Pour des prélèvements de gaz plus chauds plusieurs échangeurs peuvent être montés notamment en cascade.

L'échangeur air-gaz 6 est constitué par une calandre cylindrique verticale 13 de diamètre interne d1 munie à sa partie supérieure d'une bride circulaire 14 sur laquelle est fixée une plaque de tête 15.

La calandre 13 comporte deux ouvertures, respectivement 16 et 17, l'une 16 reliée à l'arrivée 5a des gaz industriels chargés en impuretés, l'autre 17 reliée à la sortie 5b des gaz conditionnés.

L'échangeur air-gaz 6 comporte également un tube en épingle 18 muni d'ailettes 19, formant deux branches 20 réunies par un coude 21 et dans lesquelles circule de l'air comprimé. Le tube 18 est maintenu à l'intérieur de la calandre 13 au moyen des branches 20.

Chacune desdites ailettes 19 a la forme d'un disque de diamètre d2 légèrement inférieur à d1 qui comporte une pluralité de perforations 22, dont deux d'entre elles 23 sont de plus grandes dimensions et diamétralement opposées.

Les ailettes 19 sont emmanchées sur les deux branches 20 du tube en épingle 18 au moyen des deux perforations 23 et fixées selon un pas déterminé.

Le diamètre d1 est égal, par exemple, à 76,2mm tandis que le diamètre d2 est égal à 76mm et la hauteur de la calandre 13 est de 325 mm et son diamètre extérieur est de 88,9mm.

Le tube en épingle 18 réalisé, de préférence, en inox a un diamètre de 10mm, l'entraxe des deux branches 20 étant égal, à 50mm et sa hauteur est égale à 225mm.

Les ailettes 19 sont réparties selon un pas égal, par exemple à 9mm, ce pas étant assuré par la présence sur le tube 18 d'entretoises, non représentées, placées entre deux ailettes 19 successives.

Ces entretoises servent à fixer les ailettes 19, mais lesdites ailettes peuvent également être soudées sur le tube 18.

Les perforations 22 des ailettes 19 ont des dimensions variant entre 5 et 10mm et les perforations 23 ont un diamètre égale à 10mm.

Le matériau constituant les ailettes 19 est un matériau à forte diffusivité, de préférence, de l'aluminium.

La surface d'échange ainsi réalisée est égale à 20 dcm2.

Les dimensions des ailettes 19 et de leurs perforations 22 ainsi que leur espacement sont choisis afin d'obtenir les meilleurs échanges thermiques sans que le gaz soit trop ralenti au passage dans lesdites perforations 22 et, ainsi garantir un temps de réponse du dispositif de conditionnement aussi court que possible.

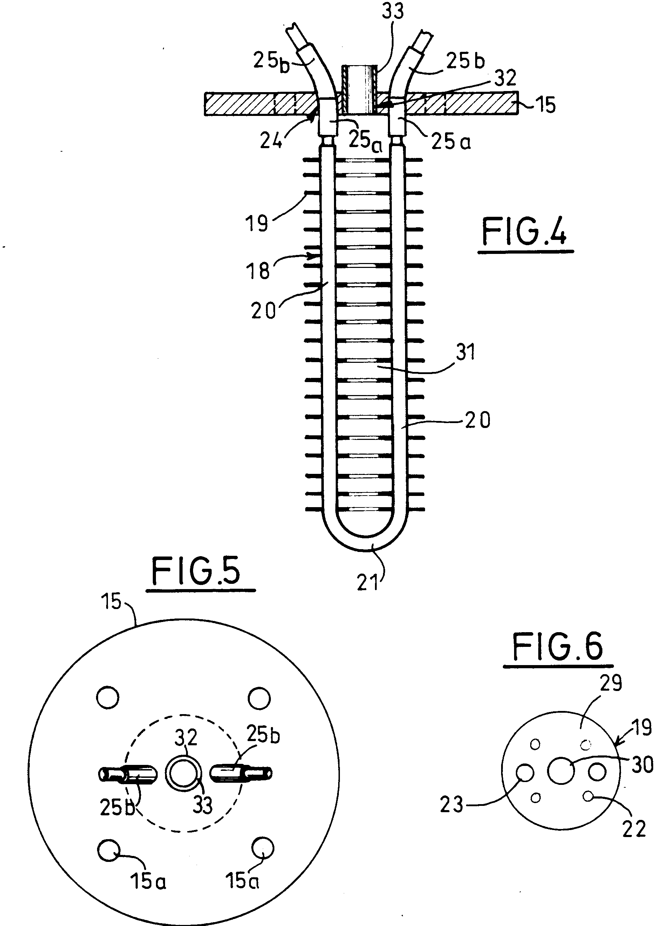

Comme représenté aux Figs. 4 et 5, la plaque de tête 15 a une forme circulaire et comporte deux orifices 24 diamétralement opposés traversant ladite plaque 15 dans son épaisseur.

Dans chacun de ces orifices 24 sont placées deux manchettes 25a et 25b, de part et d'autre de la plaque de tête 15, respectivement une manchette inférieure 25a et une manchette supérieure 25b. Les manchettes inférieures 25a sont perpendiculaires à la plaque de tête 15 et sont destinées à s'engager chacune dans une des branches 20 du tube en épingle 18.

Pour faciliter le montage, les manchettes supérieures 25b placées à l'extérieur de l'échangeur 6 et destinées à être raccordées au circuit d'air comprimé, présentent une inclinaison voisine de 30° permettant ainsi le positionnement de la sonde 7 dans ledit échangeur 6.

La plaque de tête 15 présente également un trou 32 en son milieu situé entre les deux orifices 24 et qui traverse ladite plaque 15.

Ce trou 32 permet l'introduction de la sonde 7 dans l'échangeur 6 sur le passage des gaz.

Un manchon 33 est disposé sur la face externe de la plaque 15 afin de permettre la fixation de la sonde 7 dans l'échangeur 6.

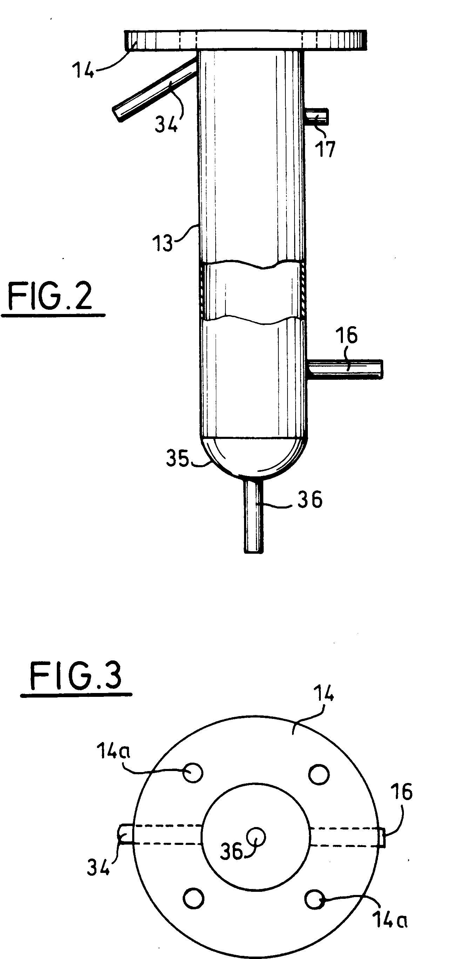

Comme représentées sur les Figs. 3 et 5, la bride circulaire 14 et la plaque de tête 15 sont munies chacune par exemple de quatre trous, respectivement 14a et 15a, régulièrement espacés sur un même diamètre, les trous 14a de la bride 14 étant en regard des trous 15a de la plaque 15 lorsque celle-ci est disposée au-dessus de la bride 14.

Au moment du montage de l'échangeur 6, on engage les manchettes inférieures 25a placées perpendiculairement sur une des deux faces planes de la plaque 15 dans les deux branches 20 du tube en épingle 18 pour solidariser ladite plaque de tête 15 et ledit tube 18.

Ensuite, on place un joint d'étanchéité 27 (Fig. 1) ayant la forme d'une couronne sur la bride 14 et l'on fixe au moyen, par exemple, de quatre boulons 28 la plaque de tête 15 sur la bride 14.

Cette caractéristique est très avantageuse car si l'on veut enlever le tube 18 de l'échangeur 6 pour quelque raison que se soit, il suffit de retirer les quatre boulons de fixation 28.

Les deux ouvertures 16 et 17 disposées sur la calandre 13, sont situées sur la périphérie de celle-ci, par exemple, dans une même section longitudinale.

L'ouverture d'arrivée 16 des gaz industriels chargés en impuretés et placée en partie basse de l'échangeur 6, sensiblement à la côte à laquelle le tube 18 forme le coude 21.

L'ouverture de sortie 17 des gaz conditionnés est placée en partie haute, par exemple, à 50mm sous la bride 14.

Selon une variante, les ailettes 19 peuvent être formées par une couronne 29 sur laquelle sont réparties de manière égale et sur un même diamètre les perforations 22, comme représentées à la Fig. 6.

La couronne 29 délimite un évidement 30 qui est un cercle de diamètre, par exemple, égal à 23 mm, légèrement supérieur au diamètre de la sonde 7.

Sur le tube 18, lorsque les ailettes 19 sont fixées, chaque évidement 30 d'une ailette 19 est aligné avec celui d'une ailette voisine, tous les évidements 30 ainsi disposés définissant un espace cylindrique 31 (Fig. 4).

Cet espace cylindrique 31 est également aligné avec le trou 32 prévu dans la plaque de tête 15.

Lorsque la sonde 7 est introduite dans l'échangeur 6, l'extrémité de ladite sonde est placée à une côte voisine de celle de la première ailette à partir du coude 21 du tube 18, dans le cas d'une sonde à dilatation de gaz dont la longueur utile est égale à 250mm.

La température ainsi mesurée est donc la température du gaz dans l'échangeur 6.

Après, affichage du point de consigne désiré qui est par exemple de +3°C pour les gaz traités aux dégoudronneurs, le régulateur de température 8 utilise l'information transmise par la sonde 7 pour commander l'ouverture de la vanne de régulation 9 et ainsi adapter le débit d'air comprimé en conséquence, ceci afin de maintenir constante la température de consigne quelles que soient les fluctuations de température et de débit du gaz à l'entrée de l'échangeur 6.

Dans le cas d'un gaz de cokerie, un pot dénaphtalineur, non représenté, peut être placé en amont de l'échangeur 6 pour écarter les risques de bouchage de l'entrée 5a du gaz.

L'échangeur de chaleur 6 est enveloppé d'un calorifuge 37 constitué par de la laine de verre (Fig. 1).

Par ailleurs, l'échangeur 6 comporte un tube 34 d'injection d'eau prévu sous la bride 14 (Figs 1 et 2).

Ce tube 34 présente un angle d'inclinaison par rapport à la verticale afin que l'eau injectée sous pression dans l'échangeur 6, en vue d'éliminer les matières provenant des impuretés du gaz, ne rencontre pas directement les ailettes 19 qui risqueraient d'être déformées.

L'angle d'inclinaison de ce tube 34 est, par exemple, égal à 45°.

Le lavage de l'échangeur 6 dure 2 à 3 minutes et s'effectue à une fréquence dépendant du débit de la composition du gaz à traiter, soit un intervalle de 15 jours à 1 an.

L'échangeur 6 comporte également un récipient 35 à sa partie basse, au fond duquel est pratiquée une ouverture 36 pour la purge des condensats.

Ce récipient 35 se présente sous la forme d'une coquille demi sphérique faisant corps avec la calandre 13 et dont le diamètre est identique à celui de ladite calandre 13.

Lorsque l'échangeur 6 est en fonctionnement, l'eau condensée, présente dans le gaz sous forme de vapeur, emporte avec elle les impuretés et est recueillie par le récipient 35 de purge des condensats.

Lors des lavages, l'eau injectée en partie haute est également récupérée dans le récipient 35.

Ce récipient 35 intégré à l'échangeur 6 évite l'emploi d'un raccord, donc écarte les risques d'entrée d'air et participe ainsi à la compacité du dispositif 1 de conditionnement, ce qui diminue, par la même, les temps de réponse de ce dispositif.

La purge des condensats et l'injection d'eau peuvent, par exemple, être associés à un système automatisé.

Dans le cas où le dispositif selon la présente invention est associé à des batteries de four à coke, on procède lors de l'analyse des fumées en vue de déterminer le degré d'avancement de la combustion, à des purges automatiques toutes les heures.

Le débit de gaz est alors de 800 l/h et on constate de manière avantageuse que le temps de réponse du conditionneur de gaz est de l'ordre de 8s.

Par contre, dans le cas où le dispositif selon la présente invention est associé à des dégoudronneurs, on se place dans les conditions les plus défavorables, car le débit de gaz est égale à 60 l/h, les purges étant alors effectuées manuellement toutes les semaines.

Le temps de réponse relevé est alors d'une centaine de secondes, ce qui correspond à celui des groupes frigorifiques classiques utilisés jusqu'à présent.

D'autre part, le dispositif 1 de conditionnement de gaz, selon l'invention, présente l'avantage du fait de sa conception, de pouvoir être sollicité en dépression et en surpression.

Ainsi, le dispositif peut aussi bien être placé en aval d'une pompe d'aspiration qu'en amont., sans risque de perturber les mesures sur les échantillons.

En prenant un gaz de cokerie dont la composition en entrée de l'échangeur 6 est :

$H_2S$ : 2,5 g/Nm3
$ClOH_8$ : 160 mg/Nm3
$NH_4^+$ : 20 mg/Nm3
t (°C) : 28°C

une analyse de sa composition, en sortie d'échangeur 6 et avant son entrée dans l'analyseur fournit les données suivantes :

$H_2S$ : 2,3 g/Nm3
$ClOH_8$ : 72 mg/Nm3
$NH_4^+$ : 18 mg/Nm3
t (°C) : 3°C

Le dispositif selon la présente invention permet donc d'effectuer un refroidissement et une purification très satisfaisantes qui procurent un gaz "propre" aux analyseurs et, par là même, contribuent de manière importante à la fiabilité des mesures ultérieures.


## Revendications

1. Dispositif (1) de conditionnement de gaz industriels chargé en impuretés, notamment de gaz de cokerie et de hauts fourneaux en vue de leur analyse, caractérisé en ce qu'il comporte :
   – une arrivée (2) d'air comprimé,
   – des moyens (3) de régulation et d'alimentation en air comprimé,
   – des moyens (4) de diffusion de l'air comprimé,
   – une arrivée (5a) de gaz industriels,
   – au moins un échangeur de chaleur air-gaz (6) pour le refroidissement et la purification des gaz industriels,
   – une sortie (5b) des gaz industriels conditionnés.

2. Dispositif selon la revendication 1, caractérisé en ce que les moyens (3) de régulation et d'air comprimé comportent une sonde (7) de mesure de la température des gaz industriels et un régulateur de température (8) relié à la sonde (7) et à une vanne de régulation (9), ladite vanne étant placée en amont des moyens (4) de diffusion de l'air comprimé.

3. Dispositif selon les revendications 1 et 2, caractérisé en ce que les moyens (3) de régulation et d'alimentation en air comprimé comportent également un filtre-détendeur (11) en amont de la sonde (7), et un filtre (12) en amont de la vanne de régulation (9), sur l'arrivée (2) d'air comprimé.

4. Dispositif selon la revendication 1, caractérisé en ce que les moyens de diffusion de l'air comprimé sont constitué par un vortex (4).

5. Dispositif selon la revendication 1, caractérisé en ce que l'échangeur de chaleur air-gaz 6 est formé par :

    – une calandre cylindrique verticale (13) de diamètre interne d1, munie à sa partie supérieure d'une bride circulaire (14) sur laquelle est fixée une plaque de tête (15), ladite calandre (13) portant deux ouvertures (16, 17), l'une (16) pour l'arrivée (5a) des gaz industriels, l'autre (17) pour la sortie (5b) des gaz conditionnés,

    – et un tube en épingle (18) muni d'ailettes (19) formant deux branches (20) réunies par un coude (21) et dans lesquelles circule l'air comprimé, ledit tube (18) étant maintenu à l'intérieur de la calandre (13) au moyen des deux branches (20), chacune desdites ailettes (19) ayant une forme de disque de diamètre d2 légèrement inférieur à d1, qui comporte une pluralité de perforations (22-23), deux (23) d'entre elles étant de plus grandes dimensions et diamétralement opposées, lesdites ailettes étant emmanchées sur les deux branches (20) au moyen desdites perforations (23) et fixées selon un pas déterminé.

6. Dispositif selon la revendication 5, caractérisé en ce que la plaque de tête (15) a une forme circulaire et comporte deux orifices (24) diamétralement opposés traversant ladite plaque (15) dans son épaisseur, chacun desdits orifices (24) reliant deux manchettes (25a, 25b) situées de part et d'autre de la plaque (15), l'une (25a) desdites manchettes étant perpendiculaire à la plaque (15) et destinée à s'engager dans une des branches (20) du tube (18).

7. Dispositif selon la revendication 5, caractérisé en ce que les ailettes (19) sont formées par une couronne (29) sur laquelle sont également réparties sur une même diamètre les perforations (22), ladite couronne (29) délimitant un évidement (30), chacun desdits évidements (30) étant aligné avec celui d'une ailette (19) immédiatement voisine et les évidements (30) définissant un espace cylindrique (31).

8. Dispositif selon les revendications 2 et 5 à 7, caractérisé en ce que la plaque de tête (15) comporte un trou central (32) aligné avec l'espace cylindrique (31) et un manchon (33) dans l'axe du trou (32), pour l'introduction et la fixation de la sonde (7) dans l'échangeur (6).

9. Dispositif selon la revendication 5, caractérisé en ce que l'échangeur (6) comporte un tube (34) d'injection d'eau aménagé sous la bride (14).

10. Dispositif selon la revendication 5, caractérisé en ce que l'échangeur (6) comporte un récipient (35) en partie basse, au fond duquel est pratiquée une ouverture (36) pour la purge des condensats.

11. Dispositif selon la revendication 5, caractérisé en ce que le tube en épingle (18) est en inox.

12. Dispositif selon la revendication 5, caractérisé en ce que les ailettes (19) sont réalisées dans un matériau à forte diffusivité thermique.

13. Dispositif selon la revendication 12, caractérisé en ce que le matériau à forte diffusivité thermique est de l'aluminium.

14. Dispositif selon la revendication 5, caractérisé en ce que l'échangeur (6) est enveloppé d'un calorifuge (37).

15. Dispositif selon la revendication 14, caractérisé en ce que le calorifuge (37) est constitué par de la laine de verre.

FIG.1

FIG.2

14
34
17
13
16
35
36

FIG.3

14
14a
16
36
34
14a

FIG.4

FIG.5

FIG.6

EP 0 506 519 A1

**Office européen des brevets**

# RAPPORT DE RECHERCHE EUROPEENNE

Numero de la demande

EP    92 40 0725

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 738 147 (TOMLIN) <br> * colonne 3, ligne 45 – colonne 5, ligne 23 * <br> * abrégé; figure 1 * <br> --- | 1-3 | G01N33/00 |
| A | US-A-4 191 541 (JENKINS) <br> * colonne 3, ligne 44 – colonne 4, ligne 3 * <br> * colonne 4, ligne 32 – colonne 6, ligne 35 * <br> * abrégé; figure 1 * <br><br> ----- | 1,4 | |
| | | | **DOMAINES TECHNIQUES RECHERCHES (Int. Cl.5)** <br><br> G01N |

Le présent rapport a été établi pour toutes les revendications

| Lieu de la recherche | Date d'achèvement de la recherche | Examinateur |
|---|---|---|
| LA HAYE | 21 JUILLET 1992 | R.A.P. BOSMA |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)

11